(19) Europäisches Patentamt
European Patent Office
Office européen des brevets

(11) Publication number: 0 144 474
B1

(12) # EUROPEAN PATENT SPECIFICATION

(45) Date of publication of patent specification: 01.04.87

(51) Int. Cl.$^4$: C 12 M 1/36, A 24 B 15/20

(21) Application number: 83307518.7

(22) Date of filing: 09.12.83

(54) **Continuous fermentation process.**

(43) Date of publication of application:
19.06.85 Bulletin 85/25

(45) Publication of the grant of the patent:
01.04.87 Bulletin 87/14

(84) Designated Contracting States:
BE CH DE FR GB LI NL

(58) References cited:
EP-A-0 024 152
EP-A-0 089 225
FR-A-1 005 902
US-A-4 097 339

CHEMICAL ABSTRACTS, vol. 87, 1977, page 455, no. 83182f, Columbus, Ohio, USA; K. ROBINSON et al.: "A comparison of pH-controlled and dissolved oxygen-controlled nutrient addition for the maintenance of steady-state in a mixed continuous culture"

(73) Proprietor: FABRIQUES DE TABAC REUNIES S.A.
Quai Jeanrenaud 3 P.O. Box 11
CH-2003 Neuchâtel-Serrières (CH)

(72) Inventor: Hofer, Michel Georges
Sous-le-Village 13
CH-2208 Les Hauts-Geneveys (CH)

(74) Representative: Bass, John Henton et al
REDDIE & GROSE 16 Theobalds Road
London WC1X 8PL (GB)

EP 0 144 474 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The present invention relates to continuous fermentation processes in which nitrogen and carbon are taken up from the fermentation medium by a microorganism growing therein, and provides a method whereby control of the process can be readily achieved and the supply of assimilable carbon during the fermentation adjusted automatically in response to changes in the fermentation medium. The invention is particularly suitable for application to processes for the continuous denitrification of nitrogen-containing tobacco material by microorganisms which are capable of metabolic assimilation of nitrogenous compounds.

During fermentation, and the accompanying assimilation of nitrogen and carbon compounds, the pH of the fermentation medium has a tendency to change for several reasons. When ammonia is the source of nitrogen, the pH tends to fall. Ammonia in solution (below pH9) exists as $NH_4^+$, and the microorganism incorporates it into the cell as $R—NH_3^+$, where R is an organic radical. In the process, a hydrogen ion is left in the medium.

If nitrate is the source of nitrogen, then hydrogen ions are removed from the medium to reduce the nitrate ion ($NO_3^-$), for example to nitrogen or $R—NH_3^+$, and the pH of the medium tends to rise. If organic amino compounds are used for growth, then the pH tends to rise as the compounds are deaminated.

When organic acids such as lactic acid, acetic acid or pyruvic acid are produced in the fermentation, pH changes also occur in the medium.

In conducting a continuous fermentation process, it is desirable to maintain the pH of the medium at a value which is optimal for the growth of the microorganism. This can be achieved by the continual addition of acid or alkali, as pH regulators, to compensate for the tendency to change. The present inventor has found that it is possible to utilize the demand of a fermentation system for acid or alkali to regulate the supply of the carbon source and thereby optimize consumption of the carbon source.

Thus, if the nitrogen source is nitrate or organic amino-compounds in a limiting substrate, it is possible to regulate the supply of the carbon source with respect to the nitrogen source by monitoring the acid consumption of the fermentation. Correspondingly, if the nitrogen source is ammonia, it is possible to regulate relate the supply of the carbon source with respect to the nitrogen source by monitoring the alkali consumption of the fermentation.

The invention is accordingly characterised in that the carbon source requirement of the fermentation is regulated by feeding the carbon source quantitatively in direct proportion to the addition of pH regulator required to maintain the pH value within a desired control range.

Control of a fermentation process can thus be achieved by a biochemical feedback control mechanism based on the optimisation of the acid or alkali consumption based on the quantitative physiology of the fermentation system. As will be appreciated, the variable acid or alkali consumption should be measured accurately and monitored continuously for the best possible control of the process.

The actual rate of addition of the carbon source as a function of the nitrogen source (in a limiting substrate) requires the determination of several ratios, more especially the ratio of the quantity of nitrogen source being fed into the fermentor to the carbon source requirement of the fermentation, that is the C:N ratio, and the ratio of the quantity of nitrogren source being fed into the fermentor to the proton or electron requirement of the fermentation. The latter ratio can be calculated theoretically, but it is preferable to determine the exact ratio experimentally for each type of fermentation.

Establishment of these two ratios enables the ratio of proton or electron requirement (acid or alkali consumption) to carbon source requirement (for example, sugar) to be determined. This in turn enables the supply of the carbon source to be directly controlled by the acid or alkali consumption, and several systems embodying this relationship can be employed.

In accordance with a first practical embodiment of the invention, the supply of acid or alkali and the supply of carbon source are separately but simultaneously regulated by a pH controller to maintain the pH within a desired range in response to information from a pH sensor in the fermentation medium. In any given fermentation system, under given conditions, the ratio between the acid or alkali supply and the carbon source supply is a constant which can be determined empirically. The pumps supply acid or alkali and carbon source solutions in a desired proportion at rates regulated by the pH controller in response to pH readings provided by the sensor.

In accordance with a second practical embodiment of the invention, and arising from the observations made in developing the first embodiment, the acid or alkali supply, together with the carbon source, are mixed in proportions corresponding to those determined empirically for the given system and conditions, and the single mixed solution is injected into the fermentation medium under the regulation of a pH controller responsive to a pH sensor.

The invention thus provides means for automatically maintaining in a continuous fermentation system those conditions and rates of supply of acid or alkali and of the carbon source that result in optimal functioning of the system. It has the further advantage that it relies solely on a pH sensor, which is a robust and reliable type of sensor, and avoids the use of sensors, such as phosphate, nitrate and glucose electrodes or gas analysers, that may prove unreliable in the long term under the strict conditions necessary for the continuous fermentation. In many processes, the latter devices only perform data acquisition and

processing but, unlike the pH sensor employed in the process of this invention, cannot be relied upon for actual control by a feedback mechanism.

The invention is not restricted to the use of a particular microorganism. However, in its application to the fermentation denitrification of tobacco materials, the preferred organisms are those which are capable of reducing nitrate by way of an assimilatory metabolic pathway. Examples of such microorganisms and their preferred strains include *Candida utilis* NCYC 707; *Candida berthetii* CBS 5452, *Candida utilis* NCYC 321, *Candida utilis* NCYC 359 and *Enterobacter aerogenes* ATCC 13048, corresponding to DSM 30053. These strains are obtainable under the stated designation number from the following depositories, identified by their abbreviations:

NCYC National Collection of Yeast Cultures, Norwich, England;

CBS Centraal Bureau voor Schimmelcultures, Baarn, Netherlands;

ATCC American Type Culture Collection, Rockville, Maryland, U.S.A.;

DSM Deutsche Sammlung von Mikroorganismmen, Goettingen, Germany.

By way of example, the application of the invention will be further explained with reference to the continuous fermentative denitration of tobacco materials containing nitrates, for example tobacco extracts, by means of the yeast *Candida utilis.*

The assimilation of nitrate by *C. utilis* proceeds by a metabolic assimilatory pathway by way of nitrite to ammonia and thence to organic nitrogen.

The formal stoichiometry of this metabolic assimilaton indicates the 8 equivalents of reducing power are consumed when one nitrate ion is reduced to the level of ammonia. The metabolic pathway employed in assimilatory denitrification can be represented as follows:

$$8(H) + H^+ + NO_3^-NH_4^+ + OH^- + 2H_2O$$

Experiments carried out with nitrate-limited continuous cultures have established that the quantity of carbon needed for the fermentation and the acid consumption are both related to the nitrate content of the tobacco material being treated.

The primary function of the acid is as a proton source to replace the hydrogen ions consumed in the reduction of nitrate. This function may be performed by either mineral acid, for example phosphoric acid, or organic acids. An organic acid that can be assimilated by the yeast, such as citric, lactic or acetic acid, has the additional advantage of being able to serve as a carbon source and thus reduce the consumption of other carbon sources, such as sugar.

The carbon source may be any material that will provide the necessary carbons in organic form which can be utilized by the microorganisms to assimilate nitrate, nitrite and the like. With the Candida yeasts, glucose, sucrose, maltose, celobiose, ethanol, glycerine or citrate are suitable carbon sources, but the carbon source may also be derived from an organic acid employed to adjust the pH of the work mixture, as explained above.

In a manually controlled operation of such a continuous fermentation process, using *C. utilis,* it was found that in normal conditions (dilution rate 0.10 to 0.2h$^{-1}$, nitrate-nitrogen content of the extract about 1 g/l), there is an optimal acid consumption, and the system works well when this is achieved. This optimal consumption corresponds to about 2% of the flow of extract through the fermentor.

Two different phenomena may be observed during the fermentation. If the amount of sugar (supplied as carbon source) with respect to the quantity of nitrate present is insufficient, then, where a mineral acid is employed for pH regulation, denitration will no longer be complete. When an organic acid capable of assimilation is employed, an increase in the acid consumption is observed. This may be explained by the fact that in this case the acid is no longer used solely as a proton source but also as a carbon source. On the other hand, an excess of sugar leads to repression of several enzymes of the respiratory chain by the surplus sugar (the so-called Pasteur countereffect, or Crabtree effect). This has the consequence of stopping cellular growth and reducing the acid consumption to almost nil.

In a manually controlled microbiological denitration process, the occurrence of these phenomena makes it necessary to keep the acid consumption under observation, and to add more sugar if the acid consumption is found to be less than the established optimum, or add less sugar if the acid consumption is above the optimum.

In acordance with the biochemical feedback principle of this invention, the supply of the carbon source is now directly linked to the supply of acid necessary to maintain a constant pH value in the fermentation medium.

In establishing a control system according to the invention for the fermentation by *C. utilis* of tobacco extracts containing nitrate, it was first necessary to determine the optimum C:N ratio, as mentioned above. Many trials were conducted, and it was found that the optimum ratio is equal to about 10:1.

The ratio of the proton requirement of the fermentation to the quantity of nitrate continuously supplied can be calculated theoretically, but it may be influenced by many factors, for example the presence of ammonia, alpha-amino nitrogen, working pH and buffer power of the medium. It was therefore preferred to determine the exact ratio experimentally under the conditions to be employed in the actual operation to be controlled. In the *C. utilis* system, a constant pH of 5.0 is preferred, and the acid:sugar ratio was determined by trials at this pH value.

As a result of experiments utilizing the automatic but separate supply of acid and sugar solutions in accordance with the first embodiment referred to above, it was discovered that the process could be more simply yet reliably operated by metering, under pH control, a single solution containing acid and sugar in the optimum ratio established by trial. The system thus obtained, which was continuously supplied with a predetermined flow of extract having a well-defined nitrate concentration, was automatically supplied with its requirement of carbon in proportion to its acid requirement.

The reduction of nitrate to ammonia in such a system requires hydrogen ions, which are removed from the medium, leading to an increase in pH. If the pH value exceeds the set point, the controller sends out a signal to actuate the acid/sugar pump to deliver the mixed solution.

In this way a biochemical feedback control has been provided, which can adapt itself to perturbations in the system.

The acid serves to bring the pH back to the set point, while the sugar is used as the requisite carbon source. If the quantity of nitrate fed into the fermentor varies, the acid consumption is automatically changed as a function of this, and so is the addition of sugar.

Experimental trials have confirmed the reliability of this system.

The invention will now be described in greater detail, by way of example only, with reference to the accompanying drawings in which:

Fig. 1 is a diagrammatic illustration of a first embodiment of the invention; and

Fig. 2 is a diagrammatic illustration of a second embodiment of the invention.

In the embodiment shown in Fig. 1, the fermentation process is conducted in a fermentor 10, which is supplied continuously with a fermentation medium, for example tobacco extract, by a pump 11. The incoming medium may be sterilised by a heat exchanger 12. The contents of the fermentation vessel are stirred and aerated by an impeller system 13, and undesirable foaming is avoided by a mechanical foam breaker 14. Treated medium leaves the system through the line 15. A pH sensor 16 is in contact with the contents of the vessel and provides a continuous record of the pH value to a pH controller 17.

The pH controller 17 develops a signal which regulates the supply pH regulator and carbon source in a pre-determined proportion from a pH regulator supply 18, which may contain acid or alkali depending on the characteristics of the fermentation being conducted, and a supply 19 of a carbon source, e.g. sugar solution by means of variable delivery pumps or control valves 21 and 22. The system provides for adjustment of the ratio between the acid or alkali and the sugar or other carbon source flow rates, so that it is adaptable to different fermentation types or conditions to provide the optimum ratio in each case.

In Fig. 2, a second embodiment is shown, in which acid and carbon source, or alkali and carbon source, are provided together from a single mixed solution cotaining these two components in proportions that are pre-determined in accordance with the fermentation type and conditions.

As in Fig. 1, the fermentation vessel 10 is supplied with fermentation medium and its pH continuously monitored by apparatus indicated by the same reference numerals as in that Figure. The pH controller 17, however, provides a signal in response to the sensed pH, which actuates a variable delivery pump 23 to deliver the mixed pH regulator/carbon source solution from a supply 24.

The following examples illustrate the operation of the process according to the invention in the apparatus described in Figs. 1 and 2.

In both examples, the fermentation process employed was the microbial assimilatory denitration of a nitrogen-containing tobacco extract obtained by extracting tobacco with water at 80°C with a tobacco:water ratio of 1:12.

The fermentation was conducted with *C. utilis* NCYC 707 at a temperature of 30.0°C and a dilution rate of $0.18h^{-1}$. The contents of the vessel were aerated at the rate of 0.15vvm (volumes air/volume of (fermentor/min)) and the working pH of the medium is maintained at 5.0.

The capacity of the fermentor was 500 kg. The extract contained 0.51 g/kg nitrate-nitrogen and 0.59 g/kg ammonia-nitrogen and was supplied to the fermentor at the rate of 80.0 kg/h, giving an input of $NO_3^-$ of 2.92 moles/h and of $NH_4^+$ 3.37 moles/h. The products of fermentation were removed at the rate of 1.16 kg/h biomass and 89.5 kg/h fermented extract. Analysis of the latter in each example showed that it contained 0.0 g/kg nitrate-nitrogen, 0.0 g/kg ammonia-nitrogen and 0.0/kg glucose.

Example 1

The fermentation was carried out in the apparatus described with reference to Fig. 1. The pH regulator was an acid in the form of lactic acid of 76% concentration supplied from one container, and 30% glucose solution was supplied from the other container.

When a working pH of 5.0 was maintained, it was found that the lactic acid solution was delivered at the rate of 1.25 kg/h and the glucose solution at the rate of 9.5 kg/h, giving an input of lactic acid of 10.56 moles/h and of glucose 15.83 moles/h.

Example 2

The fermentation operation in this case was conducted in a system as described in relation to Fig. 2. A mixed pH regulator/carbon source solution was employed, being an aqueous solution containing 10% lactic acid and 30% glucose. When a working pH of 5.0 was maintained, it was found that the mixture was supplied to the fermentor at a rate of 9.5 kg/h. This corresponds to an input of 10.56 moles/h lactic acid and 15.83 moles/h glucose. This is identical to the inputs in Example 1, which is to be expected but confirms the reliability of the systems employed.

**Claims**

1. A process for the continuous fermentation of a medium containing sources of assimilatable nitrogen and carbon, in which a pH regulator substance is added to maintain the pH value of the medium within a control range, characterised in that the carbon source requirement of the fermentation is provided by feeding the carbon source quantitatively in direct proportion to the addition of pH regulator required to maintain the pH value within the said range.

2. A process according to claim 1 characterised in that the source of carbon and the pH regulator are separately fed from respective supplies thereof in response to a signal produced by a pH sensor in contact with the fermentation medium.

3. A process according to claim 1 characterised in that the carbon source and the pH regulator are mixed together in a single solution in proportions determined to be appropriate for the fermentation being conducted, and the single solution is fed in response to a signal from a pH sensor in contact with the fermentation medium.

4. A process according to any of claims 1 to 3, characterised in that the material being fermented comprises a nitrogen-containing tobacco material, and the nitrogen source for the fermentation comprises nitrate contained in the tobacco material.

5. A process according to claim 4, characterised in that the microorganism employed for the fermentation is *Candida utilis.*

6. A process according to claim 4 or 5, characterised in that the carbon source comprises a sugar.

7. A process according to any of claims 1 to 6 characterised in that the pH of the fermentation medium is mintained within the said range of the addition of an organic acid that can be assimilated by the microorganism employed and serves as part of the carbon source.

**Patentansprüche**

1. Verfahren zur kontinuierlichen Fermentierung eines assimilierbare Stickstoff- und Kohlenstoffquellen enthaltenden Mediums, in welchem eine den pH-Wert regulierende Substanz zugegeben wird, um dem pH-Wert des Mediums innerhalb eines Kontrollbereiches zu halten, dadurch gekennzeichnet, daß das Erfordernis der Kohlenstoffquelle der Fermentierung durch quantitatives Einspeisen der Kohlenstoffquelle in direktem Verhältnis zur Zugabe des zur Aufrechterhaltung des pH-Werts in diesem Bereich benötigten pH-Regulators bereitgestellt wird.

2. Verfahren nach Anspruch 1, dadurch gekennzeichnet, daß die Kohlenstoffquelle und der pH-Regulator getrennt voneinander aus den jeweiligen Vorräten als Antwort auf ein von einem in Kontakt mit dem Fermentierungsmedium befindlichen pH-Sensor produzierten Signal eingespeist werden.

3. Verfahren nach Anspruch 1, dadurch gekennzeichnet, daß die Kohlenstoffquelle und der pH-Regulator in eine einzige Lösung in für die durchzuführende Fermentierung als geeignet bestimmten Verhältnissen zusammengemischt werden und die einzige Lösung als Antwort auf ein Signal von einem mit dem Fermentierungsmedium in Kontakt befindlichen pH-Sensor eingespeist wird.

4. Verfahren nach einem der Ansprüche 1 bis 3, dadurch gekennzeichnet, daß das Material, welches fermentiert wird, ein stickstoffhaltiges Tabakmaterial umfaßt und die Stickstoffquelle für die Fermentierung im Tabakmaterial enthaltenes Nitrat umfaßt.

5. Verfahren nach Anspruch 4, dadurch gekennzeichnet, daß der für die Fermentierung verwandte Mikroorganismus *Candida utilis* ist.

6. Verfahren nach Anspruch 4 oder 5, dadurch gekennzeichnet, daß die Kohlenstoffquelle Zucker umfaßt.

7. Verfahren nach einem der Ansrüche 1 bis 6, dadurch gekennzeichnet, daß das pH des Fermentierungsmediums durch Zugabe einer organischen Säure, welche vom verwandten Mikroorganismus assimiliert werden kann und als Teil der Kohlenstoffquelle dient, in dem Bereich gehalten wird.

**Revendications**

1. Procédé pour la fermentation en continu d'un milieu contenant des sources d'azote et de carbone assimilables, dans lequel on ajoute une substance régulatrice du pH pour maintenir la valeur du pH du milieu dans une plage requise, caractérisé en ce que la demande en source de carbone de la fermentation est assurée en fournissant la source de carbone de manière quantitative en proportion directe de l'addition de l'agent de régulation du pH requis pour maintenir la valeur du pH dans ladite plage.

2. Procédé suivant la revendication 1, caractérisé en ce que la source de carbone et l'agent de régulation du pH sont fournis séparément à partir de réserves respectives de ceux-ci en réponse à un signal produit par un détecteur de pH qui est en contact avec le millieu de fermentation.

3. Procédé suivant la revendication 1, caratérisé en ce que la source de carbone et l'agent de régulation du pH sont mélangés ensemble dans une solution unique et dans des proportions déterminées pour être appropriées à la fermentation qui est effectuée et en ce que la solution unique est fournie en réponse à un signal provenant d'un détecteur de pH qui est en contact avec le milieu de fermentation.

4. Procédé suivant l'une quelconque des revendications 1 à 3, caractérisé en ce que la matière en fermentation comprend un produit de tabac azoté et en ce que la source d'azote pour la fermentation comprend du nitrate contenu dans le produit de tabac.

5. Procédé suivant la revendication 4, caractérisé en ce que le micro-organisme utilisé pour la fermentation est *Candida utilis*.

6. Procédé suivant la revendication 4 ou 5,

caractérisé en ce que la source de carbone comprend un sucre.

7. Procédé suivant l'une quelconque des revendications 1 à 6, caractérisé en ce que le pH du milieu de fermentation est maintenu à l'intérieur de ladite plage par addition d'un acide organique qui peut être assimilé par le micro-organisme utilisé et qui forme une partie de la source de carbone.

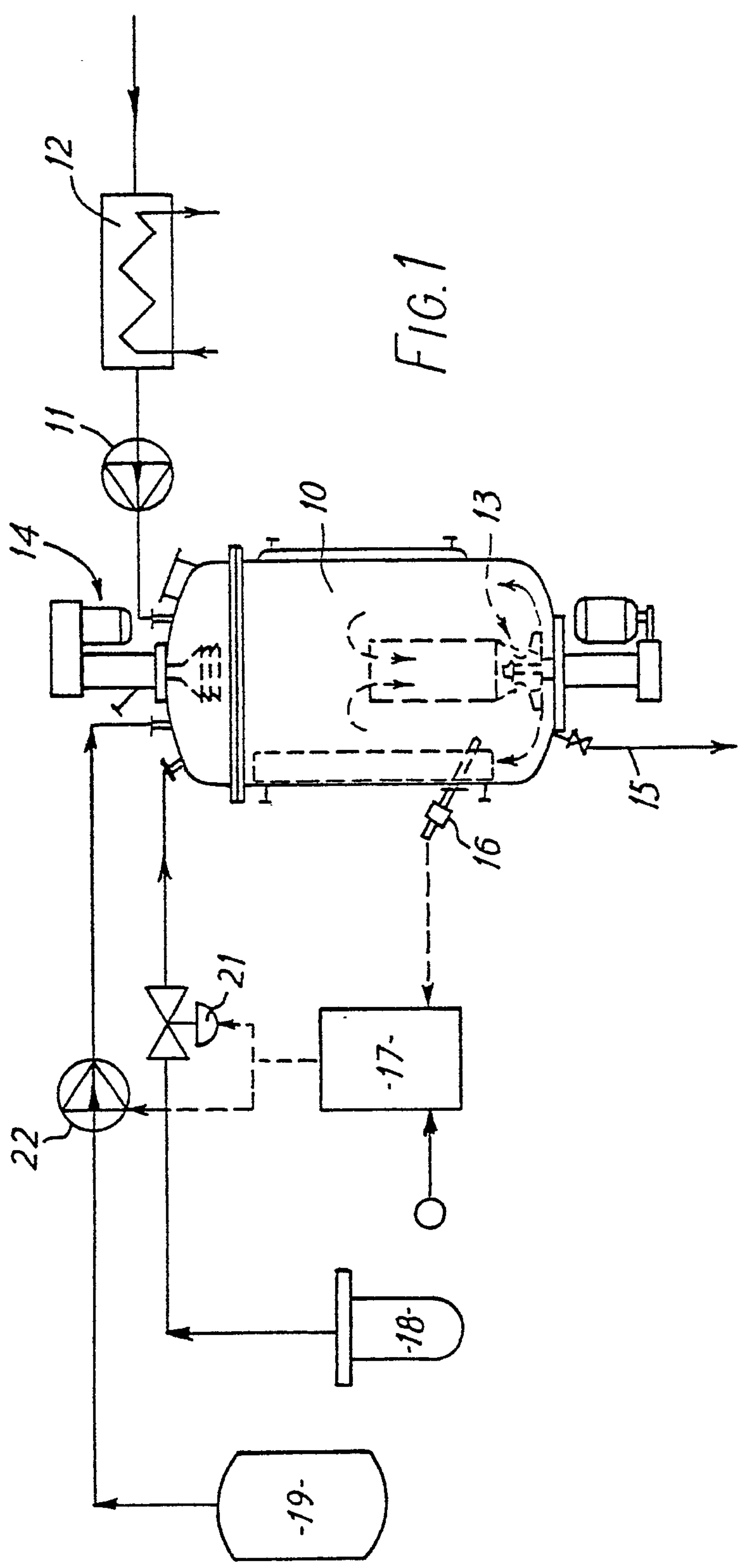


FIG.1

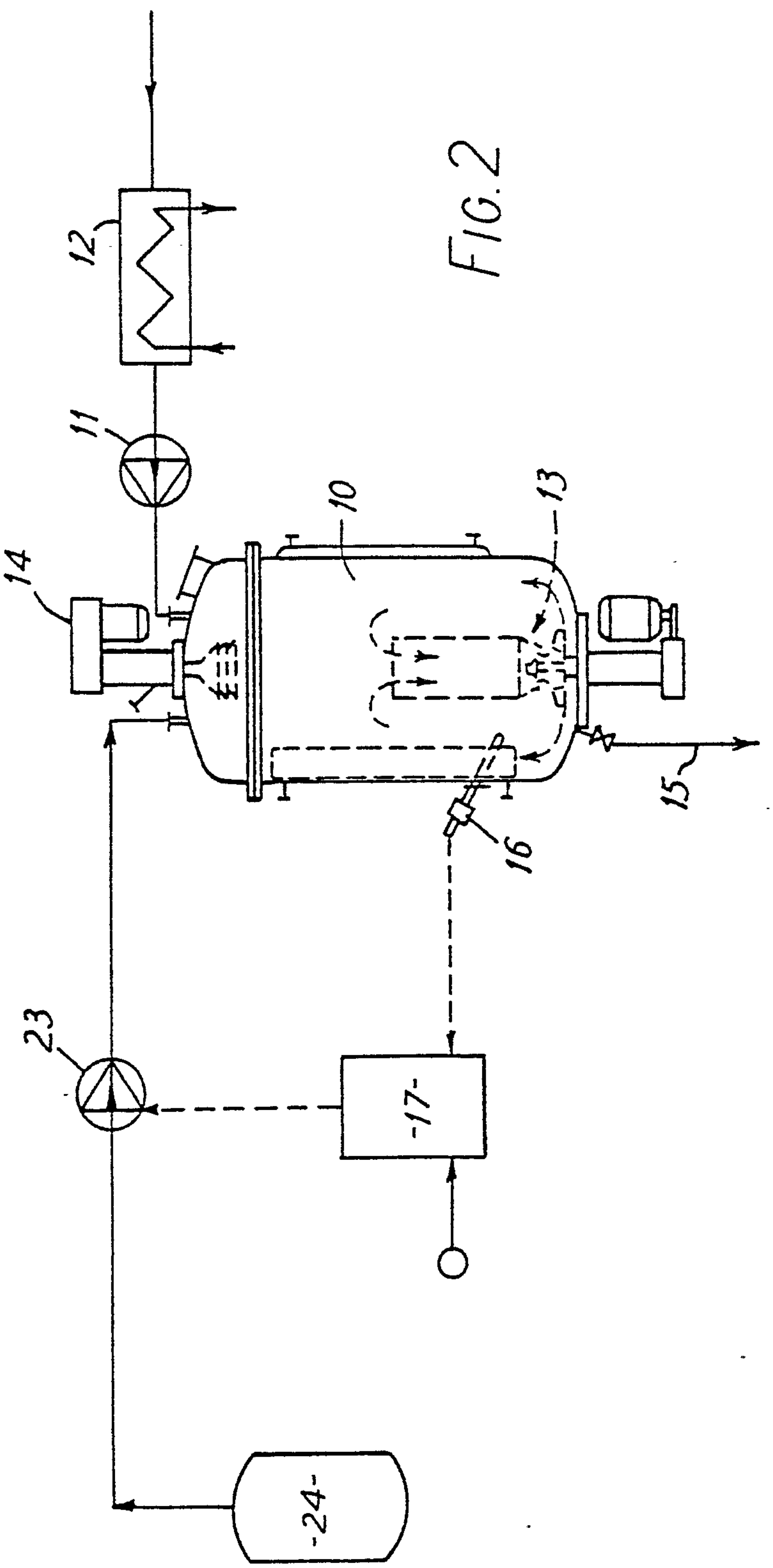


FIG. 2